# EUROPEAN PATENT APPLICATION

(11) **EP 2 692 302 A2**
(43) Date of publication of application: **05.02.2014**
(21) Application number: 12764514.1
(22) Date of filing: 29.03.2012
(51) Int. Cl.: A61B 17/34, A61B 17/29, A61M 25/01

(54) **MINIMALLY INVASIVE SURGICAL INSTRUMENT HAVING SHAFT INCLUDING INTERNAL TORQUE-TRANSMISSION MEMBER**

(30) Priority: 31.03.2011 KR 20110029771
(71) Applicant: Jeong,, Chang Wook, Seoul 135-857 (KR)
(72) Inventor: KIM, Hyung Tae, Incheon 406-720 (KR)
(74) Representative: Zardi, Marco
(86) International application number: PCT/KR2012/002340
(87) International publication number: WO 2012/134201

(57) **Abstract**

The present invention relates to a minimally invasive surgical instrument having a shaft including an internal torque transmission member. According to one aspect of the present invention, the minimally invasive surgical instrument comprises a shaft, and an end effector connected to one end of the shaft. The shaft has at least one curved portion, and the at least one curved portion can transmit torque for operating the end effector in a rolling direction by means of a torque-transmission member therein.

## Description

### FIELD OF THE INVENTION

The present invention relates to a minimally invasive surgical instrument having a shaft including an internal torque transmission member.

### BACKGROUND

Minimally invasive surgery is a surgical approach that involves the use of instruments inserted through several tiny incision openings to perform a surgery causing minimal tissue trauma in human or animal bodies.

The minimally invasive surgery relatively reduces changes in metabolism of a patient in the period of post-surgical care, so it facilitates rapid recovery of the patient. Therefore, the minimally invasive surgery shortens the length of hospitalization of the patient after the surgery and allows the patient to return to normal physical activities in a short period of time. In addition, the minimally invasive surgery causes less pain and leaves fewer scars on the patient's body after the surgery.

One of the general forms of the minimally invasive surgery is endoscopy. Among the others, a laparoscopy that involves minimally invasive inspection and operation inside abdominal cavity is known as the most general form of endoscopy. To operate a standard laparoscopic surgery, the abdomen of the patient is insufflated with gas and at least one small incision is formed to provide an entrance for laparoscopic surgical instruments, through which a trocar is inserted. When performing the surgery, it is general that a user puts the laparoscopic surgical instruments into a surgical site or the like through the trocar, and manipulates (or controls) the instruments from the outside of abdominal cavity. In general, the laparoscopic surgical instruments include a laparoscope (for observation of a surgical site) and other working tools. Herein, the working tools are similar to the conventional tools used for small incision surgery, except that the end effector or working end of each tool is separated from its handle or the like by a shaft. For instance, the working tools may include a clamp, a grasper, scissors, a stapler, a needle holder, and so forth. Meanwhile, the user monitors the procedure of the surgery through a monitor that displays the images of the surgical site which are taken by the laparoscope. The endoscopic approaches similar to the above are broadly used in retroperitoneoscopy, pelviscopy, arthroscopy, cisternoscopy, sinuscopy, hysteroscopy, nephroscopy, cystoscopy, urethroscopy, pyeloscopy, and so on.

The inventor(s) has developed various minimally invasive surgical instruments useful for the above-mentioned minimally invasive surgeries and has already disclosed the features of the structures and effects of the same in Korean Patent Application Nos. 2008-51248, 2008-61894, 2008-79126 and 2008-90560, the contents of which are incorporated herein by reference in its entirety. Additionally, the inventor(s) have also introduced a minimally invasive surgical instrument with improved functionality, which is more advantageous for users and patients, in Korean Patent Application Nos. 2010-115152 and 2011-3192, the contents of which are incorporated herein by reference in its entirety.

Herein, the inventor(s) now present a minimally invasive surgical instrument that may be more conveniently manipulated by a user while retaining the functional advantages of the minimally invasive surgical instruments disclosed in the above Korean applications.

### SUMMARY OF THE INVENTION

One object of the present invention is to solve all the above problems in prior art.

Another object of this invention is to provide a minimally invasive surgical instrument having a bent shaft wherein an end effector has good characteristics in terms of its roll direction operation.

Yet another object of this invention is to provide a minimally invasive surgical instrument wherein the collision of end effectors may be avoided when two or more minimally invasive surgical instruments are used together.

Still another object of this invention is to provide a minimally invasive surgical instrument wherein the interference of handling units may be avoided when two or more minimally invasive surgical instruments are used together.

According to one aspect of the invention to achieve the objects as described above, there is provided a minimally invasive surgical instrument comprising a shaft and an end effector being connected to one end of the shaft, wherein the shaft comprises at least one bend and the at least one bend is capable of transmitting torque to operate the end effector in a roll direction by means of a torque transmission member therein.

In addition, there may be provided other ways to implement this invention.

According to the invention, there is provided a minimally invasive surgical instrument having a bent shaft wherein an end effector has good characteristics in terms of its roll direction operation.

According to the invention, there is provided a minimally invasive surgical instrument wherein the collision of end effectors may be avoided when two or more minimally invasive surgical instruments are used together.

According to the invention, there is provided a minimally invasive surgical instrument wherein the interference of handling units may be avoided when two or more minimally invasive surgical instruments are used together.

### BRIEF DESCRIPTION OF THE DRAWINGS

Fig. 1 shows the overall appearance of a minimally invasive surgical instrument according to one embodiment of the invention.
Fig. 2 shows the interior of a first bend 131 and a second bend 132 of Fig. 1.
Fig. 3 is a cross-sectional view of a first flexible resin 133 of Fig. 2.
Fig. 4 shows the configuration of the first flexible resin 133 of Fig. 2 in detail.
Fig. 5 is an exploded view of a part of a handling unit 110 of Fig. 1.
Fig. 6 shows the interior of a part of a minimally invasive surgical instrument according to another embodiment of the invention.
Fig. 7 is a cross-sectional view of a part of a first bend 131 of Fig. 6.
Fig. 8 shows how an end effector 100 and a first rotating member 133' of Fig. 6 are connected.
Fig. 9 is an exploded view of a part of a handling unit 110 of Fig. 6.
Fig. 10 shows the interior of a part of a minimally invasive surgical instrument according to yet another embodiment of the invention.
Fig. 11 shows how an end effector 100 and an adjacent rotating member 136" of Fig. 10 are connected.
Fig. 12 is an exploded view of some components shown in Fig. 11.
Fig. 13 shows how opening/closing wires (OW) are connected in a minimally invasive surgical instrument according to an embodiment of the invention.
Fig. 14 shows the interior of a part of a minimally invasive surgical instrument according to still another embodiment of the invention.
Figs. 15 and 16 are exploded views showing how a tube unit 136□ of Fig. 14 is connected to an end effector 100 and a handling unit 110, respectively.

### DETAILED DESCRIPTION OF THE PREFERRED EMBODIMENTS

In the following detailed description of the invention, references are made to the accompanying drawings that show, by way of illustration, specific embodiments in which the invention may be practiced. These embodiments are described in sufficient detail to enable those skilled in the art to practice the invention. It is to be understood that the various embodiments of the invention, although different from each other, are not necessarily mutually exclusive. For example, specific shapes, structures, or characteristics described herein may be implemented as modified from one embodiment to another embodiment without departing from the spirit and the scope of the invention. Furthermore, it shall be understood that the locations or arrangements of individual elements within each embodiment may be also modified without departing from the spirit and the scope of the invention. Therefore, the following detailed description is not to be taken in a limiting sense, and the scope of the invention is to be taken as encompassing the scope of the appended claims and all equivalents thereof. In the drawings, like reference numerals refer to the same or similar elements throughout the several views.

Hereinafter, preferred embodiments of the present invention will be described in detail with reference to the accompanying drawings to enable those skilled in the art to easily implement the invention.

### First Embodiment

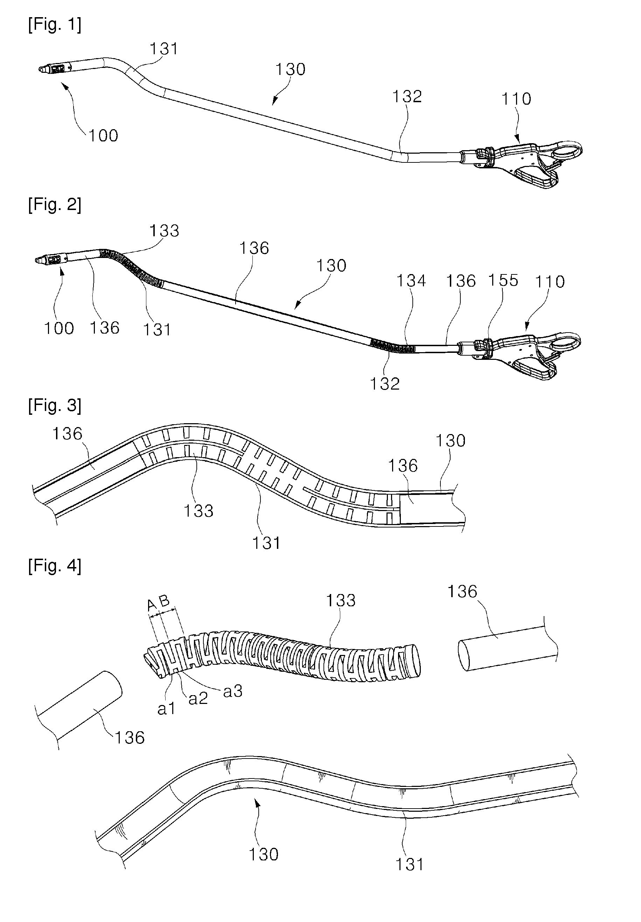

Fig. 1 shows the overall appearance of a minimally invasive surgical instrument according to one embodiment of the invention. Further, Fig. 2 shows the interior of a first bend 131 and a second bend 132 of Fig. 1

Reference will be made to Fig. 1. The minimally invasive surgical instrument according to the present embodiment may comprise an end effector 100 to perform surgery by using surgical tools (not shown) or functioning itself as a surgical tool; a handling unit 110 to control the operation of the end effector 110 according to a user's manipulation; a shaft 130 to connect the end effector 100 and the handling unit 110, wherein the end effector 100 is disposed at one end of the shaft 130 such that it may operate in a roll direction and the handling unit 110 is disposed at the other end; and a first bend 131 and a second bend 132 in the shaft 130. As shown, the first bend 131 may be disposed close to the end effector 100, and the second bend 132 may be disposed close to the handling unit 110. The first bend 131 may be formed in a curved shape as shown so that the collision or the like of the end effectors 100 may be avoided when two or more minimally invasive surgical instruments are used together. The second bend 132 may be formed in a shape spreading outwardly from the longitudinal central axis of the shaft 130 as shown, so that the interference or the like of the handling units 110 may be avoided when two or more minimally invasive surgical instruments are used together.

Reference will be made to Fig. 2. The shaft 130 may include therein a first flexible resin 133, a second flexible resin 134 and a plurality of linear members 136. (The flexible resins may be formed from Teflon or the like, and the preferably cylindrical linear members 136 may be formed from a rigid material.) The first flexible resin 133 and the second flexible resin 134 may substantially correspond to the first bend 131 and the second bend 132 of the shaft 130 located outside the flexible resins, respectively, so that they are connected between the end effector 100 and the handling unit 110 by means of the linear members 136. When the user rotates a roll sprocket 155 included in the handling unit 110 (i.e., applies torque to the roll sprocket 155), the flexible resins 133 and 134 may operate the end effector 100 in the roll direction even though the shaft 130 does not operate in the roll direction. (In this case, the flexible resins 133 and 134 and the linear members 136 may also operate in the roll direction together with the end effector 100.)

Fig. 3 is a cross-sectional view of the first flexible resin 133 of Fig. 2. Further, Fig. 4 shows the configuration of the first flexible resin 133 of Fig. 2 in detail.

Reference will be made to Figs. 3 and 4. As shown in Fig. 4, the first flexible resin 133 may have a configuration in which a bending member A bendable in a pitch direction and a bending member B bendable in a yaw direction are alternately arranged. The bending member A may be comprised of two annular members (a1 and a3) and one connecting member (a2) to connect the annular members therebetween. (The connecting member a2 may preferably be disposed on a central axis of the annular members a1 and a3.) The connecting member a2 may allow the bending member A to bend only in the pitch direction. In the same way, the bending member B may be configured to bend only in the yaw direction.

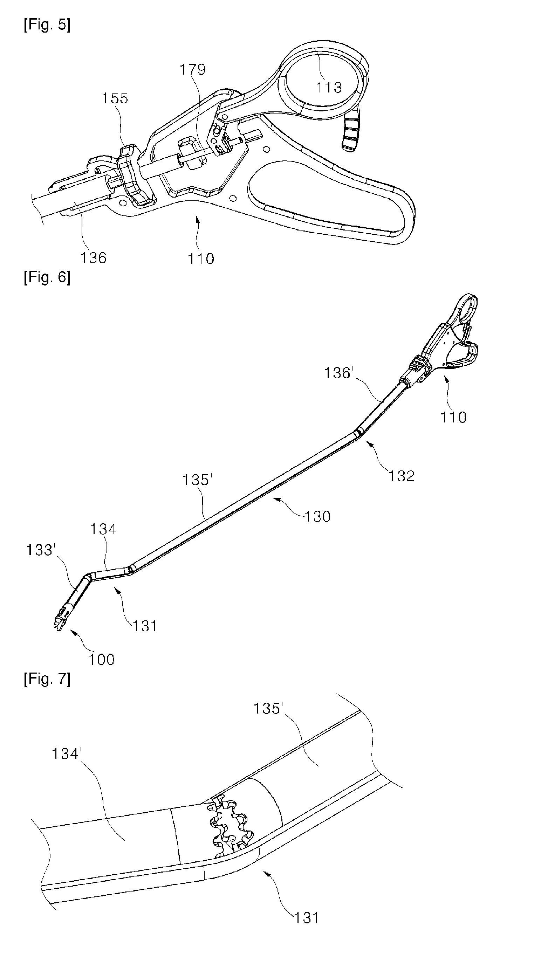

Fig. 5 is an exploded view of a part of the handling unit 110 of Fig. 1.

Reference will be made to Fig. 5. The linear members 136 are connected with the roll sprocket 155 so that when the user rotates the roll sprocket 155, the rotation may be transmitted to the second flexible resin 134, the first flexible resin 133 and the end effector 100 connected thereto. In this case, the shaft 130 does not operate in the roll direction while only the flexible resins 133 and 134 and the linear members 136 operate in the roll direction. Eventually, the end effector 100 may operate in the roll direction almost without any other operations. Meanwhile, when the user manipulates a rotatable handle 113 of the handling unit 110, the opening/closing wires 179 connected thereto are pushed or pulled to open or close the end effector 100, as disclosed in the above-mentioned Korean applications.

With regard to the various following embodiments, the configurations which are different or modified from that of the first embodiment will be mainly described below.

### Second Embodiment

Fig. 6 shows the interior of a part of a minimally invasive surgical instrument according to another embodiment of the invention.

Reference will be made to Fig. 6.

The minimally invasive surgical instrument according to the present embodiment may comprise an end effector 100, a handling unit 110, a shaft 130, a first bend 131 and a second bend 132, in the same manner as the first embodiment. According to the present embodiment, the end effector 100 may also carry out the roll direction and/or opening/closing operations.

The shaft 130 may include first to fourth rotating members 133'-136' therein. As shown, the first to fourth rotating members 133'-136' may be surrounded by the shaft 130 as they are sequentially connected from the end effector 100 to the handling unit 110. In order to achieve the unique effects of the present invention, each of the first to fourth rotating members 133'-136' may be connected to an adjacent rotating member as being tilted at an angle relative to the adjacent rotating member. The first bend 131 and the second bend 132 may be the bends in the shaft 130 resulting from such tilt.

The first to fourth rotating members 133'-136' may act together to transmit the rotation from the handling unit 110 to the end effector 100, thereby operating the end effector 100 in the roll direction. (In this case, the shaft 130 may not operate in the roll direction.) In order to act together in the above manner, the first to fourth rotating members 133'-136' may have a crown or bevel gear element at at least one of both ends thereof. At least some of the first to fourth rotating members 133'-136' may be formed from a rigid material having small torsion so as to facilitate the transmission of the rotation.

Fig. 7 is a cross-sectional view of a part of the first bend 131 of Fig. 6.

Reference will be made to Fig. 7. As shown, each of the second and third rotating members 134' and 135' may include a crown gear element at one end which obliquely engages with each other. Typically, since the crown gear elements of the second and third rotating members 134' and 135' do not engage with each other entirely but obliquely, only some cogs thereof may engage with each other. When such mechanical structure is exposed in the body of a patient, it may cause harm to the patient and also get easily damaged. Thus, each of the rotating members 133'-136' and the connecting parts thereof need to be water-tightly surrounded by the shaft 130.

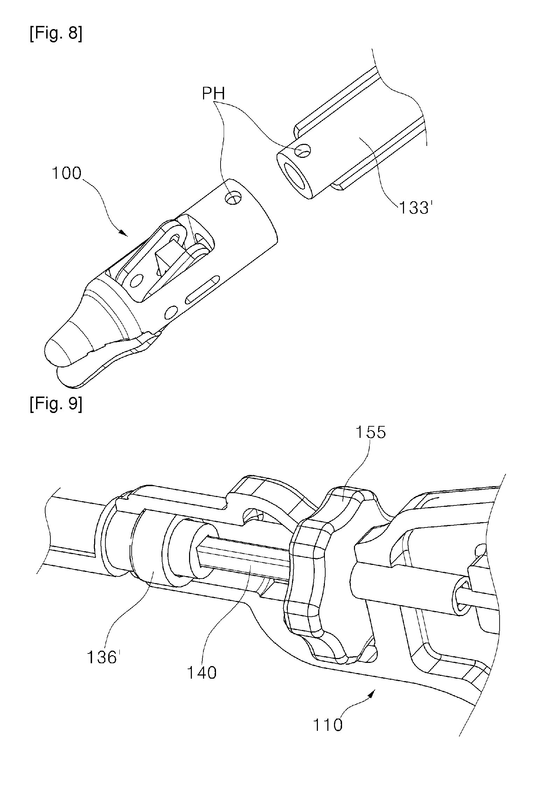

Fig. 8 shows how the end effector 100 and the first rotating member 133' of Fig. 6 are connected.

Reference will be made to Fig. 8. As shown, pin holes (PH) may be formed at one end of the end effector 100 on the opposite side of pincers for holding surgical instruments and at the opposing end of the first rotating member 133'. Therefore, the end effector 100 may be attached to or detached from the first rotating member 133' included in the shaft 130 as necessary (by means of a pin (not shown)).

Fig. 9 is an exploded view of a part of the handling unit 110 of Fig. 6.

Reference will be made to Fig. 9. As shown, the fourth rotating member 136' may be connected with the roll sprocket 155 by means of a rotator member 140. Accordingly, when the user rotates the roll sprocket 155, the rotation may be transmitted via the rotator member 140 connected thereto and further via the fourth to first rotating members 136'-133' to the end effector 100. In this case, the shaft 130 does not operate in the roll direction while only the rotating members 133'-136' and the rotator member 140 operate in the roll direction. Eventually, the end effector 100 may operate in the roll direction almost without any other operations.

Meanwhile, when the user manipulates a rotatable handle (not shown) of the handling unit 110, the opening/closing wires (not shown) connected thereto are pushed or pulled to open or close the end effector 100, in the same manner as the first embodiment. To implement such configuration, cavities may be formed within the above-mentioned rotating members 133'-136' and rotator member 140.

### Third Embodiment

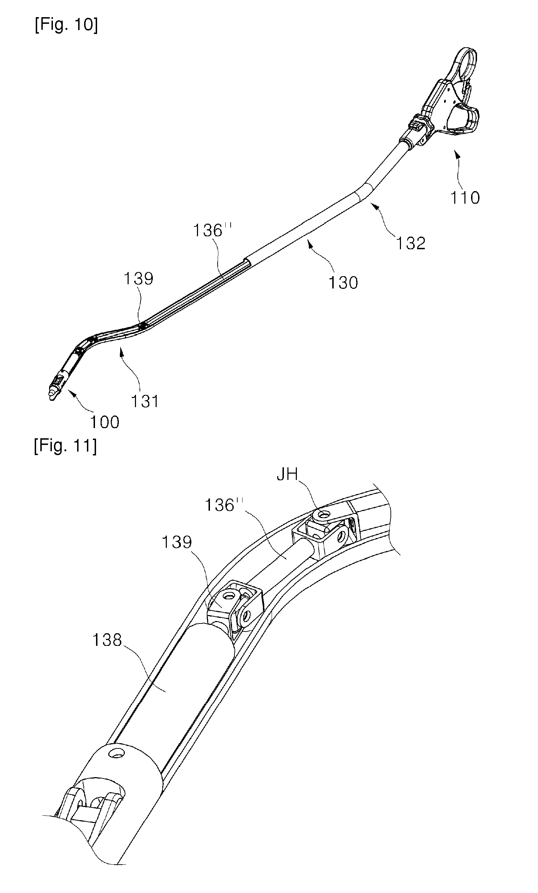

Fig. 10 shows the interior of a part of a minimally invasive surgical instrument according to yet another embodiment of the invention.

Reference will be made to Fig. 10.

The minimally invasive surgical instrument according to the present embodiment may comprise an end effector 100, a handling unit 110, a shaft 130, a first bend 131 and a second bend 132, in the same manner as the first embodiment. According to the present embodiment, the end effector 100 may also carry out the roll direction and/or opening/closing operations.

The shaft 130, particularly the first and second bends 131 and 132 may include therein a plurality of universal joints 139 and a plurality of rotating members 136" being connected to the universal joints 139 at at least one of both ends thereof. In order to achieve the unique effects of the present invention, each of the plurality of rotating members 136" may be connected to each other with a corresponding one of the universal joints 139 being interposed therebetween as illustrated, so that they have tilt angles and lengths suitable to form the first and second bends 131 and 132.

Fig. 11 shows how the end effector 100 and the adjacent rotating member 136" of Fig. 10 are connected.

Reference will be made to Fig. 11. As shown, the end effector 100 and the adjacent rotating member 136" may be connected via the universal joint 139 and the connecting member 138 fixed to the end effector 100. (In this case, there may be formed in the universal joint 139 a plurality of joint holes (JH) to be described below.) The rotating member 136" may transmit the received rotation to the end effector 100 via the universal joint 139 and the connecting member 138, almost without causing the end effector 100 to operate in the other directions. In this case, the rotation received by the rotating member 136" may be transmitted from the handling unit 110 via several other rotating members 136" in the same manner.

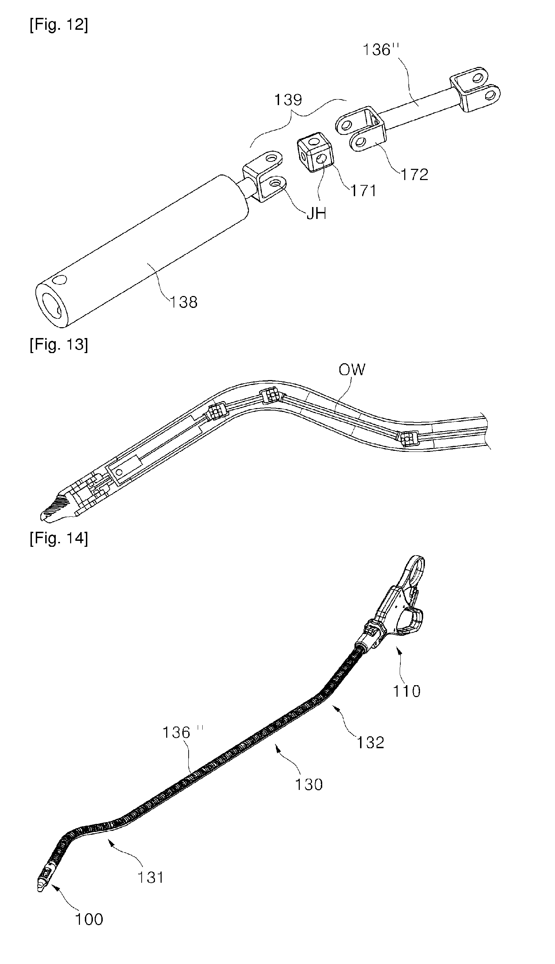

Fig. 12 is an exploded view of some components shown in Fig. 11.

Reference will be made to Fig. 12. As shown, the universal joint 139 for connecting the connecting member 138 and the rotating member 136" may comprise one cube gimbal 171 and two U-shaped members 172. The two U-shaped members 172 may be respectively disposed at the ends of the connecting member 138 and the rotating member 136" so that they are connected to each other with the one cube gimbal 171 being interposed therebetween by means of pins (not shown) being inserted into the joint holes (JH) of the cube gimbal 171 and the U-shaped members 172.

Fig. 13 shows how opening/closing wires (OW) are connected in a minimally invasive surgical instrument according to an embodiment of the invention.

Reference will be made to Figs. 12 and 13. As shown, the opening/closing wires (OW) which may perform the same function as discussed with the first embodiment may be connected from the end effector 100 to the handling unit 110 via the connecting member 138, the universal joint 139, the rotating member 136" and the like. To implement such configuration, cavities for passing the opening/closing wires (OW) may be formed within the connecting member 138 and the rotating member 136". The opening/closing wires (OW) may traverse the universal joint 139 through the joint holes (JH) thereof.

### Fourth Embodiment

Fig. 14 shows the interior of a part of a minimally invasive surgical instrument according to still another embodiment of the invention.

Reference will be made to Fig. 14.

The minimally invasive surgical instrument according to the present embodiment may comprise an end effector 100, a handling unit 110, a shaft 130, a first bend 131 and a second bend 132, in the same manner as the first embodiment. According to the present embodiment, the end effector 100 may also carry out the roll direction and/or opening/closing operations.

The shaft may include therein a long tube unit 136"' to connect the end effector 100 and the handling unit 110. The tube unit 136"' may be formed from a long strip of metallic or nonmetallic material being wound in a coil shape. In order to achieve the unique effects of the present invention, the tube unit 136"' may have a shape suitable to form the first and second bends 131 and 132.

The tube unit 136"' may transmit the rotation received from the handling unit 110 to the end effector 100 almost without causing the end effector 100 to operate in the other directions.

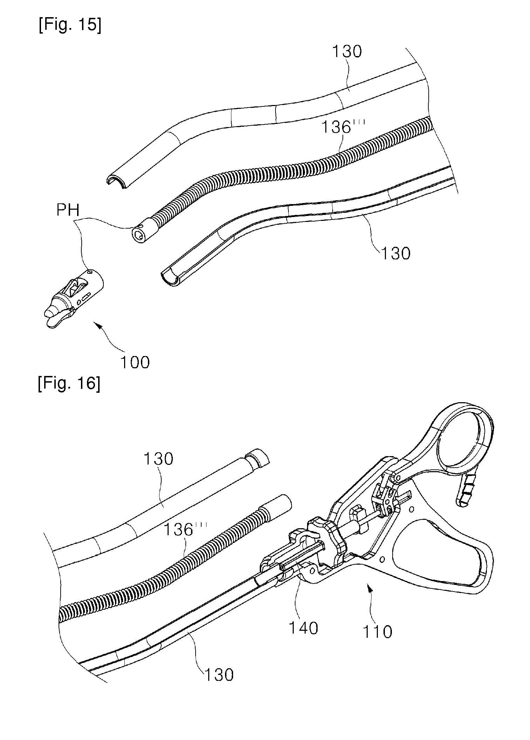

Figs. 15 and 16 are exploded views showing how the tube unit 136"' of Fig. 14 is connected to the end effector 100 and the handling unit 110, respectively.

First, as shown in Fig. 15, pin holes (PH) are formed at the end of the tube unit 136"' facing the end effector 100 so that the tube unit 136"' may be fixed to the end effector 100 by means of a pin (not shown).

Further, as shown in Fig. 16, the end of the tube unit 136"' facing the handling unit 110 may be formed so that it may be connected to a rotator member 140 included in and connected to the handling unit 110 to receive the rotation thereof. More specifically, a cavity (not shown) may be formed at the central part of the end of the tube unit 136'" facing the handling unit 110, wherein the cavity has a shape suitable to allow the tube unit 136"' to receive the above rotation from the rotator member 140. Due to the above configuration, when a rotation of the rotator member 140 is caused in the handling unit 110, the resulting torque may be transmitted to the end effector 100 via the end of the tube unit 136"' facing the handling unit 110, a coil-shaped tube of the tube unit 136'" and the end of the tube unit 136"' facing the end effector 100 to allow the end effector 100 to operate in the roll direction.

Meanwhile, it will be apparent to those skilled in the art that a cavity may also be formed in the tube unit 136'" in the present embodiment so that the opening/closing wires (not shown) may pass through the interior thereof.

Further, the present embodiment may be considered to encompass the configuration in which the above-described coil-shaped tube of the tube unit 136"' is replaced by a non-coil-shaped tube such as a known torsion tube (not shown).

### Applications

According to an application of the present invention, at least some of the components such as the handling unit 110 and the like of the minimally invasive surgical instrument may be changed or modified to those suitable to be driven by a motor-based system (not shown) such as a surgical robot, so that the minimally invasive surgical instrument may be configured to be controlled by an automatic (or semi-automatic) manipulation system rather than the user's manual manipulation.

For example, an electric motor included in a surgical robot (not shown) may operate the end effector 100 in the roll direction by directly rotating at least one of the linear members 136, rotating members 136', rotating members 136", tube unit 136"', rotator member 140 and roll sprocket 155.

For another example, the electric motor may control the opening or closing of the end effector 100 by directly pulling the opening/closing wires (OW).

For yet another example, a system may fix the rolling state or opening/closing state of the end effector 100 by controlling the drive of the electric motor.

Although the present invention has been described in terms of specific items such as detailed elements as well as the limited embodiments and the drawings, they are only provided to help general understanding of the invention, and the present invention is not limited to the above embodiments. It will be appreciated by a person of ordinary skill in the art that various modifications and changes may be made from the above description.

Therefore, the spirit of the present invention shall not be limited to the above-described embodiments, and the entire scope of the appended claims and their equivalents will fall within the scope and spirit of the invention.

## Claims

1. A minimally invasive surgical instrument comprising:
a shaft; and
an end effector being connected to one end of the shaft,
wherein the shaft comprises at least one bend; and
the at least one bend is capable of transmitting torque to operate the end effector in a roll direction by means of a torque transmission member therein.

2. A minimally invasive surgical instrument as claimed in Claim 1, wherein the at least one bend comprises a first bend formed in a curved shape facing the end effector and a second bend formed in a shape spreading outwardly from the longitudinal central axis of the shaft.

3. A minimally invasive surgical instrument as claimed in Claim 1, wherein the torque transmission member comprises a flexible resin.

4. A minimally invasive surgical instrument as claimed in Claim 1, wherein the torque transmission member comprises a plurality of rotating members; and
each of the plurality of rotating members has a gear element at at least one of both ends thereof, and is connected to an adjacent rotating member as being tilted at an angle relative to the adjacent rotating member by means of the gear element.

5. A minimally invasive surgical instrument as claimed in Claim 4, further comprising opening/closing wires being connected to the end effector, wherein the opening/closing wires are connected through cavities formed in each of the plurality of rotating members.

6. A minimally invasive surgical instrument as claimed in Claim 1, wherein the torque transmission member comprises a universal joint.

7. A minimally invasive surgical instrument as claimed in Claim 6, wherein the universal joint comprises one gimbal and two U-shaped members.

8. A minimally invasive surgical instrument as claimed in Claim 7, wherein the one gimbal and the two U-shaped members comprise joint holes, respectively.

9. A minimally invasive surgical instrument as claimed in Claim 8, further comprising opening/closing wires being connected to the end effector, wherein the opening/closing wires are connected through the joint holes.

10. A minimally invasive surgical instrument as claimed in Claim 1, wherein the torque transmission member comprises a tube unit.

11. A minimally invasive surgical instrument as claimed in Claim 10, wherein the tube unit comprises a coil-shaped tube having a curved shape.

12. A minimally invasive surgical instrument as claimed in Claim 10, wherein the tube unit comprises a non-coil-shaped tube having a curved shape.

13. A minimally invasive surgical instrument as claimed in Claim 10, further comprising opening/closing wires being connected to the end effector, wherein the opening/closing wires are connected through a cavity formed in the tube unit.
